Europäisches Patentamt

European Patent Office

Office européen des brevets

(11) Publication number: **0 428 143 A1**

## (12) EUROPEAN PATENT APPLICATION

(21) Application number: **90121733.1**

(22) Date of filing: **13.11.90**

(51) Int. Cl.5: **C12M 1/00, C12M 1/02, C12M 3/00**

(30) Priority: **16.11.89 FI 895476**

(43) Date of publication of application:
**22.05.91 Bulletin 91/21**

(84) Designated Contracting States:
**DE FR GB IT SE**

(71) Applicant: **BIODATA OY**
**Konalantie 6-8 A**
**SF-00370 Helsinki(FI)**

(72) Inventor: **Suominen, Hannu Lauri**
**Pallokuja 4 as. 14**
**SF-01280 Vantaa(FI)**

(74) Representative: **Tiedtke, Harro, Dipl.-Ing. et al**
**Patentanwaltsbüro Tiedtke-Bühling-Kinne-**
**Grupe-Pellmann-Grams-Struif Bavariaring 4**
**Postfach 20 24 03**
**W-8000 München 2(DE)**

(54) Substrate container to be used in temperature-gradient incubator.

(57) The invention relates to a substrate container to be used in a temperature-gradient incubator, which container must be sterilized and which comprises a framework (20) and a front wall (13) and a back wall (14) to be fastened thereto. On a bottom (15) of a substrate container (10), there is, between its back wall (14) and front wall (13) inside an area (11) connected to the framework (20) and forming on its part the front wall (13), a space (21), into which the condensating water can flow downward, and an actual microbic growth area (19) of the substrate container (10) is disposed above the space (21), owing to which the water flowing downward does not mix with the culture.

EP 0 428 143 A1

# SUBSTRATE CONTAINER TO BE USED IN TEMPERATURE-GRADIENT INCUBATOR

The invention relates to a substrate container to be used in a temperature-gradient incubator, which container must be sterilized and which comprises a framework and a front wall and a back wall to be fastened thereto.

Temperature-gradient incubators are used in various ways when studying the dependence of biologic phenomena on temperature. Temperature-gradient incubators are used e.g. for determining the temperatures limiting the growth of microcultures. i.e. for determining the minimum, maximum or optimum temperature of a microculture.

A previously known temperature-gradient incubator is described in the FI-patent publication 77 055. This known temperature-gradient incubator is intended to be used for studying and following the growth and other functions dependent on the temperature of the microbes. This known device is comprised of a rectangular gradient plate with a good thermal conductivity, and in connection with its longer side edges are fastened temperature-stabilization elements essentially extending along the whole length of the longer side edges of the gradient plate. In this known solution, the temperature stabilization elements are heated or cooled by means of a liquid circulation, electricity or the like, operating on the basis of an upstream principle.

In previously known temperature-gradient incubators, a container that can be closed and sterilized has been used as a substrate container, which is comprised of a rectangular metallic, glass or plastic framework, to which framework are fastened e.g. glass plates forming the front and back walls. In this known substrate container, problems have been caused by the fact that the water condensating on the free surface from the substrate flows down onto the bottom of the substrate container when using the gradient plate in a vertical position, in which case the microculture on the substrate of the container can mix by spreading and be contaminated in such a way that the reading of the result becomes more difficult.

The object of the invention is to provide a substrate container, in which the water condensating on the surface of a glass plate cannot form a problem, nor damage the culture to be grown.

For achieving the objects presented above ad subsequently, the substrate container according to the invention to be used in a temperature-gradient incubator is mainly characterized in that on the bottom of the substrate container, there is, between its back wall and front wall inside an area connected to the framework and forming on its part the front wall, a space, into which the condensating water can flow downward and that the actual microbic growth area of the substrate container is disposed above the space, owing to which the water flowing downward does not mix with the culture.

Other advantageous characteristics of the invention have further been described in the patent claims 2-4.

In the substrate container according to the invention, the water condensating on the surface of the glass plate usefully flows onto the bottom part of the container, which is not provided with a cell culture to be grown or some other culture or object to be studied.

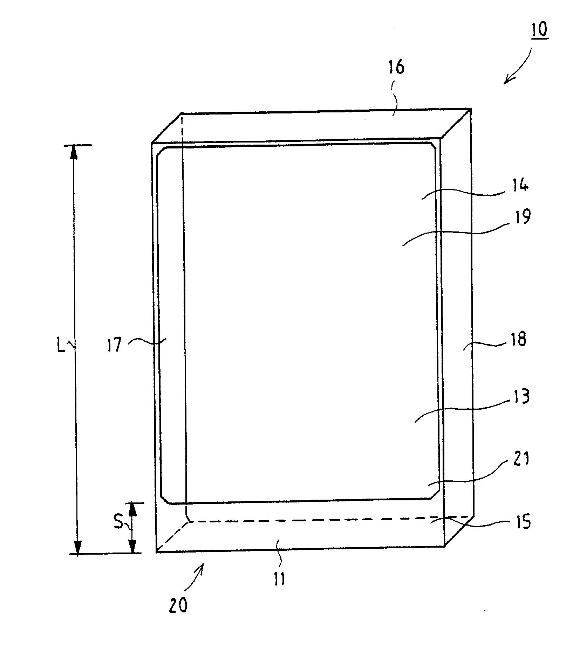

The invention is next described with reference to the figure of the accompanying drawing, which schematically shows a substrate container in accordance with the invention.

In accordance with the figure, a substrate container 10 is comprised of a framework 20 formed by upper and lower walls 16 and 15 as well as side walls 17 and 18, as well as of a front wall 13 and a back wall 14, which are transparent and made e.g. of glass. A back wall of the substrate container 10 is connected stationarily to the rectangular framework 20 formed by the walls 15,16, 17,18. In the substrate container 10, a growth area 19 is located on the back wall 14. The front wail is removably fastened to the front side of the framework 20, and before setting it in the temperature-gradient incubator, it is fixed in a sealed manner to the framework 20 e.g. by means of tape.

The substrate container 10 can be closed and sterilized e.g. for growing microcultures in the temperature-gradient incubator for studying temperature-dependent properties of microbic growth.

The front wall 13 of the substrate container 10 comprises by a distance S a substance related to the framework, i.e. an area 11. Between the front and back walls 13,14 of the substrate container 10 and inside the area 11 is formed a space 21, into which the condensating water can flow.

The areas 11 as well as the remaining framework 20 can be fabricated of non-transparent or blind material, e.g. of metal, but also other suitable materials can be utilized, such as glass or plastic.

The back wall 14 of the substrate container, which in the temperature-gradient incubator is located against the gradient plate, is made of glass, whereby the glass insulates the framework material, e.g. a metal, from the gradient plate.

When the substrate container 10 is in the temperature-gradient incubator, the water condensating on the front wall 13 of the substrate container flows down and remains on the bottom 15 of the container, i.e. on the lower wall inside the area in the space 21. In the container 10, the actual microbic substrate 19 is positioned on the back wall 14 so that the substrate 19 starts vertically from the lower edge of the container 10. i.e. from the bottom 15, and the microbic growth area by a distance S from above the bottom (15) of the the container (10). The distance S is 5-15 mm of the length L of the container 10, which length L of the container 10 is most preferably 90-100 mm. As the water thus collects on the bottom 15 of the container 10 in the space 21, the collecting water cannot damage the culture to be grown in the growth area 19.

The invention has above been described with reference to a certain embodiment example only. The space for water can also be located at some other place of the wall of the metal framework than that corresponding to the lower wall, e.g. in connection with a side wall or both side walls or lower, upper and side walls, in case the substrate container is used in several positions in the temperature- gradient incubator.

The patent claims are presented below, and many changes and modifications are possible within the inventive idea defined therein.

The invention relates to a substrate container to be used in a temperature-gradient incubator, which container must be sterilized and which comprises a framework (20) and a front wall (13) and a back wall (14) to be fastened thereto. On a bottom (15) of a substrate container (10), there is, between its back wall (14) and front wall (13) inside an area (11) connected to the framework (20) and forming on its part the front wall (13), a space (21), into which the condensating water can flow downward, and an actual microbic growth area (19) of the substrate container (10) is disposed above the space (21), owing to which the water flowing downward does not mix with the culture.

**Claims**

1. A substrate container to be used in a temperature-gradient incubator, which container must be sterilized and which comprises a framework (20) and a front wall (13) and a back wall (14) to be fastened thereto, **characterized** in that on a bottom (15) of a substrate container (10), there is, between its back wall (14) and front wall (13) inside an area (11) connected to the framework (20) and forming on its part the front wall (13), a space (21), into which the condensating water can flow downward and that an actual microbic growth area (19) of the substrate container (10) is disposed above the space (21), owing to which the water flowing downward does not mix with the culture.

2. A substrate container according to Claim 1, **characterized** in that the area (11) defining the space (21) extends by a distance (S) from the bottom (15) of the container (10), which distance (S) is 5-15 mm of the length (L) of the container (10), which length (L) is 90-100 mm.

3. A substrate container according to Claim 1 or 2, **characterized** in that the area (11) is made of framework material, glass or plastic.

4. A substrate container according to any of the Claims 1-3, **characterized** in that the back wall (14) of the substrate container (10) is made of glass.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | EP-A-0 290 722 (BIODATA) <br> – – – | | C 12 M 1/00 <br> C 12 M 1/02 <br> C 12 M 3/00 |
| A | DE-C-3 815 528 (HERAEUS) <br> – – – – – | | |
| | | | **TECHNICAL FIELDS SEARCHED (Int. Cl.5)** |
| | | | C 12 M |

The present search report has been drawn up for all claims

| Place of search | Date of completion of search | Examiner |
|---|---|---|
| The Hague | 27 February 91 | TURMO Y BLANCO C.E. |